# EUROPEAN PATENT SPECIFICATION

(11) **EP 3 423 428 B1**
(45) Date of publication and mention of the grant of the patent: **24.07.2024**
(21) Application number: 16892859.6
(22) Date of filing: 01.03.2016
(51) Int. Cl.: C07C 50/36, C07C 46/00, C07C 46/10

(54) **SYSTEMS AND METHODS FOR PRODUCING SYNTHETIC HYPERICIN**
SYSTEME UND VERFAHREN ZUR HERSTELLUNG VON SYNTHETISCHEM HYPERICIN
SYSTÈMES ET PROCÉDÉS POUR PRODUIRE DE L'HYPÉRICINE SYNTHÉTIQUE

(43) Date of publication of application: 09.01.2019
(73) Proprietor: Soligenix, Inc., Princeton, NJ 08540 (US); Arumugham, Rasappa, Princeton, NJ 08540 (US); Schaber, Christopher, Princeton, NJ 08540 (US); Rauter, Holger, 36103 Flieden (DE); Werner, Silvia, 63796 Kahl (DE)
(72) Inventor: ARUMUGHAM, Rasappa, 63796 Kahl (US); SCHABER, Christopher, 63796 Kahl (US); RAUTER, Holger, 36103 Flieden (DE); WERNER, Silvia, 63796 Kahl (DE)
(74) Representative: Winter, Brandl - Partnerschaft mbB
(86) International application number: PCT/US2016/020189
(87) International publication number: WO 2017/151111

(56) References cited:
- EP-A2- 0 432 496
- WO-A1-93/15607
- US-A- 5 543 016
- US-A1- 2012 245 392
- US-A1- 2012 245 392
- EVGENY I. KAPINUS ET AL: "Spectroscopic Investigation of the Molecular Structure of Hypericin and its Salts", MONATSHEFTE FÜR CHEMIE = CHEMICAL MONTHLY, vol. 130, no. 5, 1 May 1999 (1999-05-01), Vienna, pages 623 - 635, XP055414218, ISSN: 0026-9247, DOI: 10.1007/PL00010243
- KAPINUS ET AL.: "Spectroscopic Investigation of the Molecular Structure of Hypericin and its Salts", MONATSHEFTE FUER CHEMIE, vol. 130, 1999, pages 623 - 635, XP055414218

## Description

### FIELD OF THE INVENTION

The present invention relates to methods for producing synthetic hypericin Particularly, the present invention relates to improved methods for producing synthetic hypericin at high volume and high purity.

### BACKGROUND OF THE INVENTION

Hypericin is a natural compound found in stems and petals of plants of the genus *hypericum.* Within this genus are eight families and 43 species, including the common St.

John's wort plant, *Hypericum perforatum.* Hypericin is the principle phototoxic agent in St. John's wort. The chemical name is 1,3,4,6,8,13-hexahydroxy-10,11-dimethylphenanthro[1,10,9,8-*opqra*]perylene-7,14-dione (other names: 4,5,7,4',5',7' hexahydroxy-2,2'-dimethyl-meso naphthodianthrone; Phenanthro[1,10,9,8-opqra]perylene-7,14-dione, 1,3,4,6,8,13-hexahydroxy-10,11-dimethyl-), a compound composed of eight conjugated rings containing six hydroxyl groups, two carbonyl and two methyl groups in a symmetric pattern when inverted about a central axis.

Hypericin is one of the most important phenanthoperylene quinones extracted mainly from plants of the genus *Hypericum.* Widespread attention to the antiviral and anti-tumor properties of hypericin has spurred investigations of the chemical synthesis and biosynthesis of this unique compound. However, the synthetic strategies are challenging for organic and biological chemists.

In the past, isolation of hypericin from plants was not practical on a large scale because it requires a lengthy procedure involving extraction with large columns of solvents and cumbersome chromatographic separations on silica gel columns. The main difficulty in obtaining hypericin in a pure state from the plant material resides in its separation from the accompanying pseudohypericin. This necessitates the aforementioned chromatography with the elution of a large number of fractions, only a few of which contain the pure desired material. The concentration of hypericin in the plants is very low, not more than 0.3% based on the dry plant material.

US patent 8,629,302 B2 issued to Tobia et al discloses a method for making hypericin comprising steps starting from emodine to emodine anthrone which is then dimerized to protohypericin salt and then photoconverted to hypericin. However, the method requires recirculation of protohypericin solution to effectively convert to hypericin making the process unscalable. Furthermore, the purified hypericin was hygroscopic, adsorbing moisture over storage time and found to be in a mixture of salt and acid form. Presently, there is a need for novel and highly effective systems and methods for producing synthetic hypericin having a well-defined compositional matter on a large scale.

US 2012/245392 A1 discloses a method for preparing hypericin by converting protohypericin to a protohypericin salt and then irradiating the protohypericin salt with visible light to form hypericin.

A process for preparing hypericin is further described in EP 0 432 496 A2 by oxidative dimerization of emodin anthrone and conversion of the intermediate protohypericin to hypericin by irradiation with visible light.

The molecular structure of hypericin and its salts is discussed by EVGENY I. KAPINUS ET AL: "Spectroscopic Investigation of the Molecular Structure of Hypericin and its Salts", MONATSHEFTE FÜR CHEMIE = CHEMICAL MONTHLY, vol. 130, no. 5, 1 May 1999, pages 623-635, based on UV/Vis, IR and NMR spectra of hypericin as well of its salts.

WO 93/15607 A1 is directed to novel compounds possessing a hypericin moiety consisting of ion pairs having anti-viral activity.

### SUMMARY OF THE INVENTION

An object of the present invention provides a synthetic hypericin comprising hypericin as a mono-sodium salt in the form of a trihydrate.

These objects are solved by providing a hypericin synthesized and purified by a process comprising the following steps:
(a) photoconverting protohypericin to crude hypericin using a micro-reactor equipped with a Light Emitting Diode (LED) light source either as batch or continuous mode;
(b) treating crude hypericin with at least one salt forming reagent in a solvent at a pre-precipitation temperature; filtering the treated hypericin while providing at least one cooling ramp to reach a post-precipitation temperature, wherein a precipitate is formed; washing and filtering the precipitate in a washing solvent, and
(c) drying of the purified hypericin product with nitrogen, water vapor or air under vacuum,
wherein the purified hypericin product is a mono-sodium salt in the form of a trihydrate having the following structure

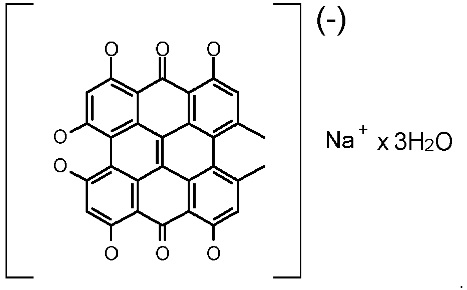

According to the present invention is further provided a method of synthesizing and purifying hypericin, the method comprising:
(a) photoconverting protohypericin to crude hypericin using a continuous flow micro-reactor;
(b) treating the crude hypericin with at least one salt forming reagent in a solvent at a pre-precipitation temperature;
(c) filtering the crude hypericin from step (b) while providing at least one cooling ramp to reach a post-precipitation temperature, wherein a precipitate is formed;
(d) washing and filtering the precipitate from step (c) with a washing solvent; and
(e) drying of the product of step (d) with nitrogen, water vapor or air under vacuum,
wherein the purified hypericin product is a mono-sodium salt in the form of a trihydrate having the following structure

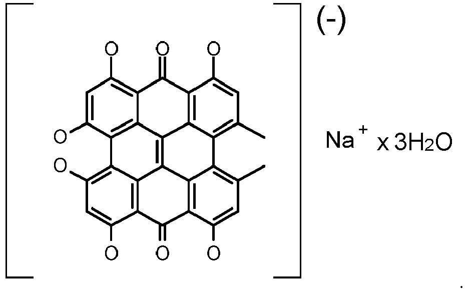

Yet another object of the present invention provides a method of preparing a purified synthetic hypericin wherein the irradiation is the result of exposure to standard light. Yet another object of the present invention provides a method of preparing a purified synthetic hypericin wherein the irradiation is the result of exposure to light. As light sources one can select suitable lamps such as a low or medium pressure mercury lamp. More preferred light sources are LED lights which can provide irradiation with a narrow desirable wavelength distribution.

In the method of the present invention it is preferred to use a LED light having a wavelength between 350-700 nm.

According to a preferred embodiment of the present invention, the hypericin yield is at least 30% of the dry product. In a further preferred embodiment the synthetic hypericin yield is at least 50% of the dry product. In a more preferred embodiment the synthetic hypericin yield is at least 70% of the dry product. In a most preferred embodiment the synthetic hypericin yield is at least 75 to 100% of the dry product.

According to a preferred embodiment of the present invention, the synthetic hypericin purity is at least 80% of the dry product. In a further preferred embodiment the synthetic hypericin purity is at least 90% of the dry product. In a more preferred embodiment the synthetic hypericin purity is at least 95% of the dry product. In a most preferred embodiment the synthetic hypericin purity is at least 97% of the dry product.

### BRIEF DESCRIPTION OF THE FIGURES

The novel features of this invention, as well as the invention itself, both as to its structure, reaction scheme and its operation, will be best understood from the accompanying drawings, taken in conjunction with the accompanying description, in which similar reference characters refer to similar parts, and in which:
Figure 1 depicts a reaction scheme for hypericin manufacture.
Figure 2 depicts a process flow diagram for the manufacture of hypericin.

### DETAILED DESCRIPTION OF THE INVENTION

The present invention comprises one or more of the following steps:
(1) irradiation of protohypericin to crude hypericin; (2) purification of crude hypericin in methanol with sodium bicarbonate; (3) drying of purified hypericin in the presence of air under reduced pressure; and (4) drying of purified hypericin in the presence of air and / or water vapor under reduced pressure.

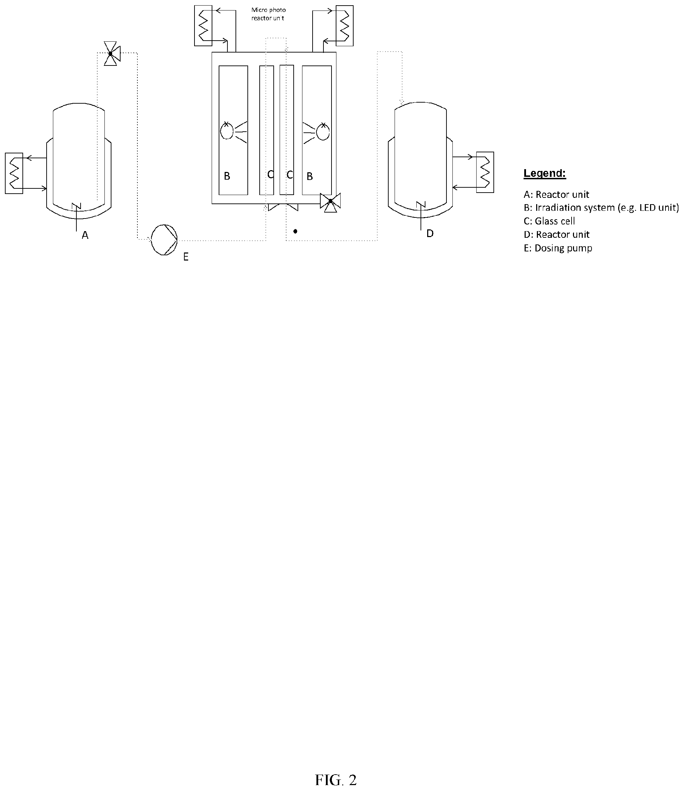

The Micro reactor system (Figure 2) used for the irradiation step consists of 2 quartz glass laminar flow cells (size: 200 mm/120 mm/40 mm; gap: ID 80 µm; volume: 1.2 ml volume) with LED panels, having the preferred parameters of 54 W light energy at designated wavelength; panel size: 175 mm/100 mm,) as shown in Figure 2.

Protohypericin was photoconverted to synthetic hypericin according to the methods presented in the Examples section. Briefly, protohypericin was mixed with acetone in a double-jacketed glass vessel equipped with a heating and cooling system. The resulting solution was filtered using a glass filter and washed with acetone. Additional acetone was added to the filtrate solution and which then was irradiated at the specified wavelength with LED panels. Irradiated solution was placed in a rotary evaporator for distillation followed by the addition of methanol for further distillation.

The distilled slurry was filtered and the solid was washed with a hexane/ethyl acetate mixture and dried. The resulting crude hypericin was mixed with methanol in a double-jacketed glass vessel equipped with a heating and cooling system and maintained at constant temperature. Next, the vessel was cooled and held at constant temperature while mixing. Crystallized hypericin was filtered and washed again with a hexane/ethyl acetate mixture and dried under nitrogen followed by vacuum drying. This drying process is also optimized for the generation of anhydrous hypericin salt.

Crude hypericin was mixed with methanol and sodium bicarbonate in a double-jacketed glass vessel equipped with a heating and cooling system and maintained at a constant pre-precipitation temperature and the resulting solution was filtered. Next, the contents were systematically cooled down to post-precipitation temperature allowing the product to be crystallized. Crystallized hypericin was filtered and washed again with a hexane/ethyl acetate mixture prior to drying under an air stream.

### EXAMPLES

### Synthetic Hypericin

### Example 1: Irradiation step of Protohypericin into Crude Hypericin:

Protohypericin (45g) was charged into a vessel followed by the addition of acetone and NaHCO₃. The suspension was heated to >40°C. The warm solution was filtered and washed with acetone.

Acetone was added to the protohypericin solution and cooled down to a temperature of 2°C. The solution was pumped through the irradiation unit at a flow rate of >15 ml/min and irradiated. The obtained hypericin solution was concentrated on a rotary evaporator at ambient temperature. Methanol was added, further distilled and concentrated. The final slurry was filtered and washed with a mixture containing hexane and ethyl acetate. The solid was dried under nitrogen followed by vacuum at room temperature. The resulting yield of crude hypericin was 27 g or roughly 60%.

Purity by HPLC [area%]: 98.12

### Example 2: Purification of Hypericin (Method step being not part of the present invention):

Crude hypericin (42g) was suspended in methanol. The suspension was mixed, heated to pre-precipitation temperature >40°C and filtered. The filtrate was cooled to post-precipitation temperature. The suspension containing purified hypericin solid was filtered and the filter cake was washed with a mixture of hexane and ethyl acetate in portions. The solid was dried in a nitrogen stream under vacuum for >48 hours. The obtained yield of pure hypericin was 40.1g or roughly 95.5%.
Purity by HPLC [area%]: 97.95
Sodium content [wt%]: 3.8
Water content [wt%]: 0.96

### Example 3: Purification of Hypericin (Method step according to the present invention):

Crude hypericin (280.4g) and methanol were charged in a vessel followed by the addition of sodium bicarbonate. The suspension was mixed and heated to pre-precipitation temperature >45 °C. The warm solution was filtered. The filtrate was transferred to a glass reactor and cooled to post-precipitation temperature allowing the hypericin to crystallize. The crystallized hypericin was filtered and the filter cake was washed with a mixture of hexane and ethyl acetate in portions. The solid was dried in air stream under vacuum. The obtained yield of pure hypericin was 212.6g or roughly 75.8%.
Purity by HPLC [area%]: 99.62
Sodium content [wt%]: 3.94
Water content [wt%]: 7.85

In order to utilize the purified hypericin as the active pharmaceutical ingredient for pharmaceutical drug applications, the present invention described in the examples above contains specific steps undertaken to reduce the residual solvents present in the final purified hypericin. Residual solvents are generally removed by drying the active pharmaceutical ingredient similar to the method of Example 2. These drying processes also remove water molecules from the active pharmaceutical ingredient resulting in the anhydrous status of the drug substance.

Chemical entities such as hypericin are not stable in their anhydrous forms and they tend to become hydrated over time even if they are protected from air by inert gas (such as nitrogen) replacement techniques. Therefore solvent removal drying procedures in the presence of air and/or water under reduced pressure have been employed to remove residual solvents while preserving the hydrated stable form of hypericin.

Accordingly the present invention provides methods to dry active pharmaceutical ingredients to reduce the residual solvents to an acceptable specification limits while keeping these drug substances in their most stable form.

### Example 4: Drying of purified hypericin (Method 1):

The preferred method utilizes the same filtration unit used for the last step of the purified final crystallized hypericin from Example 3. After the product was filtered through the unit via the use of a vacuum pump, the unit was then allowed to intake a continuous air stream through disposable sterile 0.22 micron filter on one end of the unit while the vacuum was applied to the other end of the filtration device. The residual solvents from the product were removed by adjusting the temperature of the drying unit under vacuum. The temperature was set to be in the range between 0 to 100°C, preferably 20 to 70°C and suitably 25 to 60°C. The vacuum was set in the range of 20 mbar to the atmospheric pressure, preferably 100 mbar to 700 mbar, suitably 200-600 mbar.

**Table 1: Analytical Test Results for the hypericin batch produced under Examples 3 and 4**

| Parameter Studied | Test Results |
|---|---|
| Extinction Coefficient [at 590 nm] | 51025 |

| **Related substances** [at 590 nm] | |
|---|---|
| Protohypericin [area % ] | < 0.05 |
| Impurity RRT 1.95 [area %] | 0.21 |
| Any other unspecified impurity [area %] | 0.17 |
| Total impurities [area %] | 0.38 |
| **Hypericin purity [area%]** [at 590 nm] | 99.62 |
| **Hypericin Assay [wt. %]** [at 590 nm] (anhydrous and solvent free substance) | 95.7 |

| **Residual Solvents** | |
|---|---|
| Acetone [ppm] | < 100 |
| Ethyl Acetate [ppm] | 470 |
| Hexane [ppm] | 3110 |
| Methanol [ppm] | < 100 |
| Piperidine [ppm] | 199 |
| Pyridine [ppm] | < 100 |
| Total residual solvents [wt. %] | 0.38 |
| **Water [wt. %]** | 7.85 |

| **Elemental impurities** | |
|---|---|
| Sodium content [%] | 3.94 |
| Iron content [ppm] | < 100 |
| Tin content [ppm] | < 2000 |

### Example 5: Drying of purified hypericin (Method 2):

An alternate method used vacuum oven to dry the drug substance in the presence of water vapor supplied through air intake or by placing a container of water in the oven. The residual solvents from the product were removed by adjusting the temperature of the drying unit under vacuum. The temperature was set to be in the range between 0 to 100°C, preferably 20 to 70°C and suitably 25 to 60°C. The vacuum was set in the range of 20 mbar to the atmospheric pressure, preferably 100 mbar to 700 mbar, suitable 200-600 mbar

### Solvent content (ppm) results of the dried sample

| | |
|---|---|
| Methanol | 2009 |
| Hexane | 1421 |
| Ethyl acetate | 5828 |

### Composition of purified hypericin:

A systematic study was performed to determine the water content of the purified historical batch A along with recently manufactured batches B and C and their short term stability on the water content. The samples were taken from containers that were kept closed and sealed under nitrogen and containers that were kept open to the atmosphere for the indicated time and analyzed for the water content. Historical batch A had a water content of 9.3% and did not significantly increase after exposure to the air. The recently purified batches (B and C) started with low water content (< 1%) and their values increased substantially suggesting that the hypericin molecule is hygroscopic and gets stabilized in its hydrated form. Historical batch A appeared to be stabilized at 9.3% which corresponds to the trihydrated form of the hypericin molecule (see below for calculation).

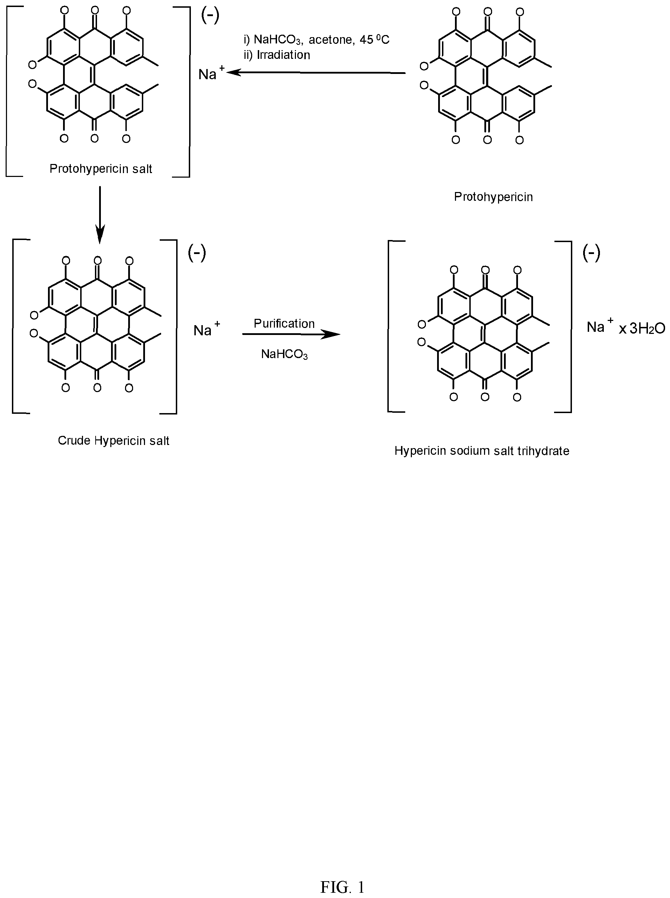

The sodium content of the manufactured batches was also monitored. During the manufacturing process, all protohypericin sub-batches were treated with sodium bicarbonate prior to the irradiation step (Figure 1). However, manufacturing of a fully substituted sodium salt (monosodium) form of the hypericin was not possible by the pre-treatment of protohypericin with sodium bicarbonate alone. Further treatment of crude hypericin with sodium bicarbonate was necessary for the quantitative yield of hypericin monosodium salt. In order to ensure that the hypericin batch was manufactured as a monosodium salt, the final purification and crystallization of the crude hypericin was performed in the presence of methanol and sodium bicarbonate. The sodium content of the recently manufactured batch following the examples in 3 and 4 was found to be 3.94% supporting the manufacture of hypericin monosodium salt in its trihydrated form.

From the above observations, calculations were made to determine the molecular composition of the hypericin related forms. Examples were shown to manufacture anhydrous as well as hydrated forms of hypericin. Also included were the examples of manufacturing of monosodium salt as well as partially sodium substituted salt forms of hypericin. Also disclosed are methods/ processes of choice to synthesize and purify hypericin in a variety of related forms such as the examples below:
Hypericin molecular weight C₃₀H₁₆O₈
Hypericin monosodium C₃₀H₁₅O₈Na
Hypericin monosodium trihydrate C₃₀H₁₅O₈Na.3H₂O
Hypericin monosodium trihydrate C₃₀H₁₅O₈Na.3H₂O with a theoretical water content of 9.3% and sodium content of 3.96%

These and other advantages of the present invention will be apparent to those skilled in the art from the foregoing specification. Accordingly, it will be recognized by those skilled in the art that changes or modifications may be made to the above-described embodiments without departing from the broad inventive concepts of the invention. For example, the precipitation and salt conversion experiments performed in methanol in the presence of sodium bicarbonate can be substituted by other solvents of 2 carbon to 5 carbon alcohol; ketones, preferably acetone, methyl ethyl ketone); alkyl acetates, preferably ethyl acetate for the solubilization step. The final washing step can be performed with solvents selected from the group consisting of mixture of ethyl acetate/ hexane (or pentane); ethers preferably methyl tertiary butyl ether (MTBE).

## Claims

1. A hypericin synthesized and purified by a process comprising the following steps:
(a) photoconverting protohypericin to crude hypericin using a micro-reactor comprising a LED light source either as batch or continuous mode;
(b) treating crude hypericin with at least one salt forming reagent in a solvent at a pre-precipitation temperature, filtering the treated hypericin while providing at least one cooling ramp to reach a post-precipitation temperature, wherein a precipitate is formed and washing and filtering the precipitate in a washing solvent, and
(c) drying of the purified hypericin product with nitrogen, water vapor or air under vacuum,
wherein the purified hypericin product is a mono-sodium salt in the form of a trihydrate having the following structure

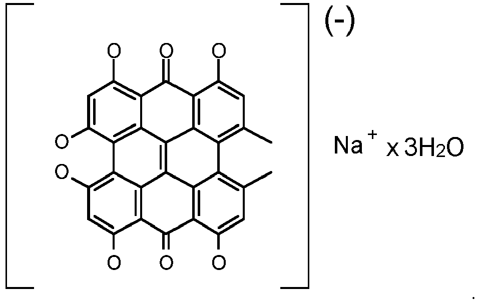

2. The hypericin of claim 1, wherein the solvent is selected from the group consisting of methanol, acetone, methyl ethyl ketone and ethyl acetate.

3. The hypericin of claim 1, wherein the washing solvent is selected from at least one of the group consisting of ethyl acetate, hexane, pentane, ether and methyl tertiary butyl ether (MTBE).

4. The hypericin of claim 1, wherein the at least one salt forming reagent is sodium bicarbonate.

5. A method of synthesizing and purifying hypericin, the method comprising:
(a) photoconverting protohypericin to crude hypericin using a continuous flow micro-reactor;
(b) treating the crude hypericin with at least one salt forming reagent in a solvent at a pre-precipitation temperature;
(c) filtering the crude hypericin from step (b) while providing at least one cooling ramp to reach a post-precipitation temperature, wherein a precipitate is formed;
(d) washing and filtering the precipitate from step (c) with a washing solvent; and
(e) drying of the product of step (d) with nitrogen, water vapor or air under vacuum
wherein the purified hypericin product is a mono-sodium salt in the form of a trihydrate having the following structure

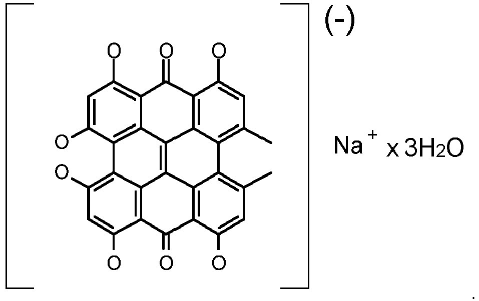

6. The method of claim 5, wherein the photoconverting results from irradiation with LED light having a wavelength between 350-700 nm.

7. The method of claim 5 or 6, wherein the pre-precipitation temperature is greater than 40°C and the post-precipitation temperature is less than 40°C.

8. The method of any one of claims 5 to 7, wherein the hypericin yield is at least 30%, preferably at least 50%, more preferably at least 70% and most preferably at least 75% of the dry product.

## Patentansprüche

1. Hypericin, das durch ein Verfahren synthetisiert und gereinigt wird, das die folgenden Schritte umfasst:
(a) Photokonvertieren von Protohypericin zu rohem Hypericin unter Verwendung eines Mikroreaktors, der eine LED-Lichtquelle umfasst, entweder in einem Chargen- oder kontinuierlichen Modus;
(b) Behandeln des rohem Hypericins mit mindestens einem salzbildenden Reagens in einem Lösungsmittel bei einer Vorfällungstemperatur, Filtrieren des behandelten Hypericins, während mindestens eine Kühlrampe bereitgestellt wird, um eine Nachfällungstemperatur zu erreichen, wobei ein Niederschlag gebildet wird, und Waschen und Filtrieren des Niederschlags in einem Waschlösungsmittel, und
(c) Trocknen des gereinigten Hypericinprodukts mit Stickstoff, Wasserdampf oder Luft unter Vakuum,
wobei das gereinigte Hypericinprodukt ein Mononatriumsalz in Form eines Trihydrats mit der folgenden Struktur ist:

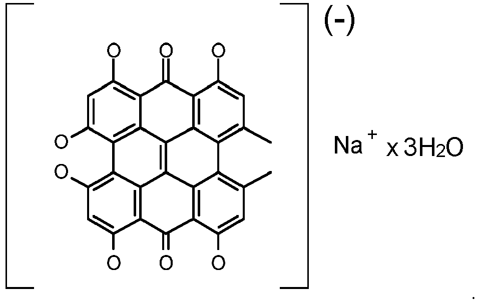

2. Hypericin nach Anspruch 1, wobei das Lösungsmittel ausgewählt ist aus der Gruppe bestehend aus Methanol, Aceton, Methylethylketon und Ethylacetat.

3. Hypericin nach Anspruch 1, wobei das Waschlösungsmittel ausgewählt ist aus mindestens einem aus der Gruppe bestehend aus Ethylacetat, Hexan, Pentan, Ether und Methyl-tert-butylether (MTBE).

4. Hypericin nach Anspruch 1, wobei das mindestens eine salzbildende Reagens Natriumbicarbonat ist.

5. Verfahren zum Synthetisieren und Reinigen von Hypericin, wobei das Verfahren umfasst:
(a) Photokonvertieren von Protohypericin zu rohem Hypericin unter Verwendung eines Mikroreaktors mit kontinuierlichem Durchfluss;
(b) Behandeln des rohen Hypericins mit mindestens einem salzbildenden Reagens in einem Lösungsmittel bei einer Vorfällungstemperatur;
(c) Filtrieren des rohen Hypericins aus Schritt (b), während mindestens eine Kühlrampe bereitgestellt wird, um eine Nachfällungstemperatur zu erreichen, wobei ein Niederschlag gebildet wird;
(d) Waschen und Filtrieren des Niederschlags aus Schritt (c) mit einem Waschlösungsmittel; und
(e) Trocknen des Produkts aus Schritt (d) mit Stickstoff, Wasserdampf oder Luft unter Vakuum,
wobei das gereinigte Hypericinprodukt ein Mononatriumsalz in Form eines Trihydrats mit der folgenden Struktur ist:

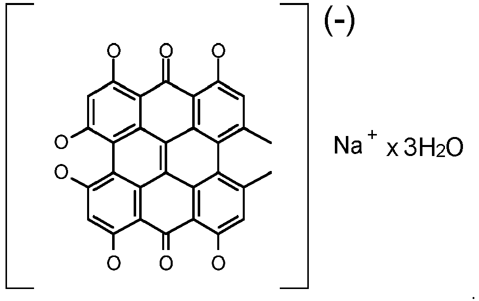

6. Verfahren nach Anspruch 5, wobei das Photokonvertieren aus einer Bestrahlung mit LED-Licht mit einer Wellenlänge zwischen 350-700 nm resultiert.

7. Verfahren nach Anspruch 5 oder 6, wobei die Vorfällungstemperatur größer als 40 °C ist und die Nachfällungstemperatur kleiner als 40 °C ist.

8. Verfahren nach einem der Ansprüche 5 bis 7, wobei die Hypericinausbeute mindestens 30 %, vorzugsweise mindestens 50 %, mehr bevorzugt mindestens 70 % und am meisten bevorzugt mindestens 75 % des Trockenprodukts beträgt.

## Revendications

1. Hypéricine synthétisée et purifiée par un processus comprenant les étapes consistant à :
(a) réaliser la photoconversion de la protohypéricine en hypéricine brute en utilisant un micro-réacteur comprenant une source de lumière à LED en mode discontinu ou continu ;
(b) traiter l'hypéricine brute avec au moins un réactif formant un sel dans un solvant à une température de pré-précipitation, filtrer l'hypéricine traitée tout en prévoyant au moins une rampe de refroidissement pour atteindre une température de post-précipitation, dans laquelle un précipité est formé et laver et filtrer le précipité dans un solvant de lavage, et
(c) sécher le produit d'hypéricine purifiée avec de l'azote, de la vapeur d'eau ou de l'air sous vide,
dans lequel le produit d'hypéricine purifiée est un sel monosodique sous la forme d'un trihydrate présentant la structure suivante

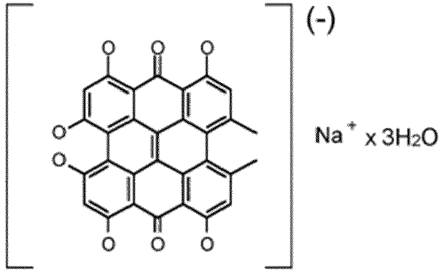

2. Hypéricine selon la revendication 1, dans laquelle le solvant est choisi dans le groupe consistant en du méthanol, de l'acétone, de la méthyléthylcétone et de l'acétate d'éthyle.

3. Hypéricine selon la revendication 1, dans laquelle le solvant de lavage est choisi parmi au moins l'un du groupe consistant en l'acétate d'éthyle, l'hexane, le pentane, l'éther et l'éther méthyltertiobutylique (MTBE).

4. Hypéricine selon la revendication 1, dans laquelle le au moins un réactif formant un sel est le bicarbonate de sodium.

5. Procédé de synthèse et de purification de l'hypéricine, le procédé comprenant les étapes consistant à :
(a) réaliser la photoconversion de la protohypéricine en hypéricine brute à l'aide d'un microréacteur à flux continu ;
(b) traiter l'hypéricine brute avec au moins un réactif formant un sel dans un solvant à une température de pré-précipitation ;
(c) filtrer l'hypéricine brute issue de l'étape (b) tout en prévoyant au moins une rampe de refroidissement pour atteindre une température de post-précipitation, dans lequel un précipité est formé ;
(d) laver et filtrer le précipité issu de l'étape (c) avec un solvant de lavage ; et
(e) sécher le produit de l'étape (d) avec de l'azote, de la vapeur d'eau ou de l'air sous vide
dans lequel le produit d'hypéricine purifiée est un sel monosodique sous la forme d'un trihydrate présentant la structure suivante

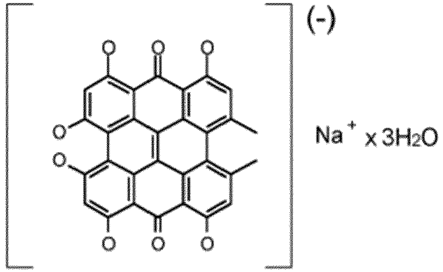

6. Procédé selon la revendication 5, dans lequel la photoconversion résulte d'une irradiation avec une lumière LED présentant une longueur d'onde comprise entre 350 et 700 nm.

7. Procédé selon la revendication 5 ou 6, dans lequel la température de pré-précipitation est supérieure à 40 °C et la température de post-précipitation est inférieure à 40 °C.

8. Procédé selon l'une quelconque des revendications 5 à 7, dans lequel le rendement en hypéricine est d'au moins 30 %, de préférence d'au moins 50 %, plus préférentiellement d'au moins 70 % et le plus préférentiellement d'au moins 75 % du produit sec.
